# EUROPÄISCHE PATENTSCHRIFT

(11) **EP 3 417 788 B1**
(45) Veröffentlichungstag und Bekanntmachung des Hinweises auf die Patenterteilung: **25.03.2020**
(21) Anmeldenummer: 17177191.8
(22) Anmeldetag: 21.06.2017
(51) Int. Cl.: A61B 6/03, A61B 6/00, A61B 5/055

(54) **GANTRY FÜR EINE MEDIZINISCHE BILDGEBUNGSVORRICHTUNG UND VERFAHREN ZUR LUFTKÜHLUNG ZUMINDEST EINER KOMPONENTE DER GANTRY**
GANTRY FOR A MEDICAL IMAGING DEVICE AND METHOD FOR AIR-COOLING AT LEAST ONE COMPONENT OF THE GANTRY
PORTIQUE POUR UN DISPOSITIF D'IMAGERIE MÉDICALE ET PROCÉDÉ DE REFROIDISSEMENT PAR AIR D'AU MOINS UN COMPOSANT DE PORTIQUE

(43) Veröffentlichungstag der Anmeldung: 26.12.2018
(73) Patentinhaber: Siemens Healthcare GmbH, 91052 Erlangen (DE)
(72) Erfinder: Krug, Rita, 90762 Fürth (DE)

(56) Entgegenhaltungen:
- DE-A1-102007 037 313
- US-A1- 2015 320 376
- US-A1- 2016 235 378
- US-B1- 6 289 073

## Beschreibung

Die Erfindung betrifft eine Gantry für eine medizinische Bildgebungsvorrichtung, eine medizinische Bildgebungsvorrichtung, eine Anordnung und ein Verfahren zur Luftkühlung zumindest einer Komponente einer Gantry einer medizinischen Bildgebungsvorrichtung.

Computertomographiegeräte können beispielsweise mit Luft gekühlt werden. Dabei gibt es einerseits die Möglichkeit, die Luft in einem geschlossenen Kreislauf in der Gantry zirkulieren zu lassen. Dabei zirkuliert die Luft in einem geschlossenen Kreislauf und wird durch einen Rückkühler, beispielsweise Wasser, rückgekühlt. Andererseits kann über Öffnungen in einer Verkleidung des Computertomographiegeräts (CT-Geräts) die Luft aus dem Untersuchungsraum zu Kühlzwecken angesaugt und aufgewärmt wieder an diesen abgegeben werden.

In einem Interventions- bzw. OP-Raum liegen meist bestimmte Luftströmungsverhältnisse vor, die insbesondere gemäß den Lehren der Reinraumtechnik ausgebildet sind. Von der Decke bis zum Boden fließt dabei Luft um den Patienten herum und bildet so eine keimfreie Umgebung in Patientennähe aus. Diese Luftströmung ist gleichgerichtet, hat beispielsweise eine Strömungsgeschwindigkeit zwischen 0,2 und 0,3 Metern pro Sekunde, ist überwiegend laminar und wird im Folgenden turbulenzarme Verdrängungsströmung, abgekürzt TAV, genannt. Dem Fachmann ist die TAV auch unter den Begriffen Laminar Flow und laminarer Luftstrom bekannt.

Die TAV darf nicht durch die Kühlung des CT-Geräts gestört werden. Je nach Ausstattung des Raumes, in dem das CT-Gerät aufgestellt ist, und Anwendung des CT-Geräts ist ein relativ großes Feld mit TAV vorgesehen, welches bei einer üblichen Größe von drei Metern mal drei Metern beispielsweise auch einen OP-Instrumententisch umfassen kann, oder ein relativ kleines Feld vorgesehen, das sich im Wesentlichen auf den OP-Bereich am Patienten beschränkt.

Befindet sich nun das CT-Gerät nicht außerhalb des TAV-Feldes, sondern innerhalb oder nur zum Teil in diesem Feld, dann findet an den Oberflächen des CT-Geräts eine Strömungsverdrängung und Umlenkung statt. Dabei bildet das CT-Gerät ein Strömungshindernis. Außerdem wird durch das Ansaugen und Ausblasen der Luft an den jeweiligen Oberflächen der Verkleidung des CT-Geräts die TAV-Strömung ebenfalls beeinflusst. Mögliche Folgen davon sind beispielsweise eine erhöhte Strömungsgeschwindigkeit, die insbesondere auf die Verdrängungswirkung des CT-Geräts als Körper in der Strömung zurückgeführt werden kann, eine erhöhte Strömungsumlenkung sowie ein verstärkter Strömungsabriss, der insbesondere auf eine ungünstige Außenkontur des CT-Geräts zurückgeführt werden kann. Alle drei Effekte führen zur Störung der TAV durch Turbulenzen.

US 6988827 B2 offenbart ein Kühlsystem für eine Gantry eines CT-Geräts.

US 7374338 B2 offenbart ein Kühlsystem für eine Gantry eines CT-Geräts.

US 7410295 B2 offenbart ein Kühlsystem für eine Gantry eines CT-Geräts.

US 2014/0378817 A1 offenbart ein Kühlsystem für ein CT-Gerät, bei dem die Lufteintrittsöffnung und die Luftaustrittsöffnungen relativ weit unten angeordnet sind, wobei die aus dem CT-Gerät austretende Luft mittels der Luftaustrittsöffnung unterhalb eines für chirurgische Eingriffe sterilen Feldes gerichtet wird. Auf Grund der Verdrängungswirkung des CT-Geräts kann es dabei jedoch zu einer signifikanten Störung der TAV kommen.

US 2015/0320376 A1 offenbart eine Gantry für eine medizinische Bildgebungsvorrichtung, wobei in einer Außenfläche der Gantry eine Lufteinströmfläche für eine Luftkühlung zumindest einer Komponente der Gantry ausgebildet ist, wobei die Gantry in einem Bereich der Lufteinströmfläche durch die Lufteinströmfläche nach oben begrenzt ist.

Für die Anwendung im OP-Bereich werden oft wassergekühlte CT-Geräte verwendet, mit denen ein Luftaustausch mit dem Raum vermieden werden kann. Allerdings kann das Mitführen der Wasserschläuche eine freie Bewegung im Raum einschränken.

Die Erfindung hat die Aufgabe, eine Luftkühlung einer Gantry einer medizinischen Bildgebungsvorrichtung bereitzustellen, welche die Raumluft, welche die Gantry umgibt, verwendet, und in Bezug auf die Luftströmungsverhältnisse verbessert ist. Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft eine Gantry für eine medizinische Bildgebungsvorrichtung, wobei in einer Außenfläche der Gantry eine Lufteinströmfläche für eine Luftkühlung zumindest einer Komponente der Gantry ausgebildet ist, wobei in einem Betriebszustand der Gantry die Gantry in einem Bereich der Lufteinströmfläche durch die Lufteinströmfläche nach oben begrenzt ist, dadurch gekennzeichnet, dass die Gantry einen stationären Tragrahmen und einen Kipprahmen aufweist, wobei die Außenfläche in einem Bereich des Tragrahmens eine Tragrahmen-Außenfläche aufweist, wobei die Außenfläche in einem Bereich des Kipprahmens eine Kipprahmen-Außenfläche aufweist, wobei eine Tragrahmen-Teilfläche der Lufteinströmfläche in der Tragrahmen-Außenfläche ausgebildet ist und/oder wobei eine Kipprahmen-Teilfläche der Lufteinströmfläche in der Kipprahmen-Außenfläche ausgebildet ist.

Insbesondere kann in dem Betriebszustand der Gantry die Gantry im Wesentlichen durch die Lufteinströmfläche nach oben begrenzt sein.

Insbesondere kann in einer Orthogonalprojektion von oben die Lufteinströmfläche wenigstens 50 Prozent, insbesondere wenigstens 70 Prozent, bevorzugt wenigstens 90 Prozent, der Außenfläche der Gantry bilden.

Insbesondere kann die Gantry einen Kühlkanal für die Luftkühlung der zumindest einen Komponente der Gantry aufweisen, wobei die Lufteinströmfläche in dem Betriebszustand der Gantry derart ausgebildet ist, dass ein insbesondere vertikal von oben auf die Gantry strömender laminarer Luftstrom durch die Lufteinströmfläche turbulenzarm in den Kühlkanal einströmen kann.

Insbesondere kann die Lufteinströmfläche für eine mittlere Lufteinströmgeschwindigkeit von 0,2 bis 0,3 Metern pro Sekunde ausgebildet sein.

Insbesondere kann eine flächige Verteilung eines Lufteinströmparameters in der Lufteinströmfläche derart an eine Neigung der Außenfläche der Gantry angepasst sein, dass der Lufteinströmparameter in einer Orthogonalprojektion von oben eine im Wesentlichen gleichmäßige flächige Verteilung aufweist.

Insbesondere kann die Lufteinströmfläche ein Lufteinströmelement aufweist, welches aus der Gruppe gewählt sein, welche aus einem Luftfilter, einer Lochblende, einer Einströmlamelle und Kombinationen davon besteht.

Insbesondere kann der Lufteinströmparameter aus der Lufteinströmparametergruppe gewählt sein, welche aus einem Druckverlustbeiwert, einem Öffnungsabstand, einer Öffnungsfläche und Kombinationen davon besteht.

Insbesondere kann die Gantry eine Luftausströmfläche für die Luftkühlung der zumindest einen Komponente der Gantry aufweisen, wobei die Luftausströmfläche und die Lufteinströmfläche miteinander mittels eines Kühlkanals verbunden sind.

Die Erfindung betrifft ferner eine medizinische Bildgebungsvorrichtung, aufweisend eine Gantry gemäß einem der in dieser Anmeldung offenbarten Aspekte.

Die Erfindung betrifft ferner eine Anordnung, aufweisend eine Gantry gemäß einem der in dieser Anmeldung offenbarten Aspekte und eine Luftstromerzeugungseinheit.

Insbesondere kann sich die Gantry in einem Betriebszustand der Gantry befinden, in dem die Gantry in dem Bereich der Lufteinströmfläche durch die Lufteinströmfläche nach oben begrenzt ist.

Insbesondere kann die Luftstromerzeugungseinheit zur Erzeugung des laminaren Luftstroms derart ausgebildet sein, dass zumindest ein Gantry-Teilluftstrom des laminaren Luftstroms von oben auf die Gantry strömt und durch die Lufteinströmfläche in die Gantry hineinströmt.

Insbesondere kann die Anordnung ferner eine Patientenlagerungsvorrichtung der medizinischen Bildgebungsvorrichtung aufweisen.

Insbesondere kann die Luftstromerzeugungseinheit zur Erzeugung des laminaren Luftstroms derart ausgebildet sein, dass zumindest ein Patienten-Teilluftstrom des laminaren Luftstroms von oben auf die Patientenlagerungsvorrichtung strömt.

Insbesondere kann die Anordnung ferner die medizinische Bildgebungsvorrichtung aufweisen.

Die Erfindung betrifft ferner ein Verfahren zur Luftkühlung zumindest einer Komponente einer erfindungsgemäßen Gantry einer medizinischen Bildgebungsvorrichtung, wobei das Verfahren die folgenden Schritte umfasst:
- Erzeugen eines laminaren Luftstroms, wobei zumindest ein Gantry-Teilluftstrom des laminaren Luftstroms von oben auf die Gantry strömt,
- Aufnahme des zumindest einen Gantry-Teilluftstroms in einen Kühlkanal der Gantry über eine Lufteinströmfläche, welche an einer Außenfläche der Gantry ausgebildet ist und welche die Gantry in einem Bereich der Lufteinströmfläche nach oben begrenzt, wobei ein Kühlluftstrom erzeugt wird, welcher durch den Kühlkanal strömt,
- Kühlen der zumindest einen Komponente der Gantry mittels des Kühlluftstroms.

Insbesondere kann die Lufteinströmfläche mehrere Luftfilter mit unterschiedlichen Druckverlustbeiwerten aufweisen. Insbesondere kann die Lufteinströmfläche mehrere Öffnungen, beispielsweise in Form von Löchern und/oder Spalten aufweisen.

Bei dem Öffnungsabstand kann es sich beispielsweise um den Abstand zwischen Löchern und/oder Spalten handeln. Bei der Öffnungsfläche kann es sich beispielweise um eine Querschnittsfläche eines Lochs und/oder eines Spalts handeln. Insbesondere kann die Lufteinströmfläche mehrere Lochbleche mit unterschiedlichen Lochabständen aufweisen. Insbesondere kann die Lufteinströmfläche mehrere Einströmlamellen mit unterschiedlicher Spaltbreite zwischen unmittelbar benachbarten Einströmlamellen aufweisen.

Die Lufteinströmfläche kann insbesondere als eine zusammenhängende Fläche ausgebildet sein oder mehrere voneinander separierte Teil-Lufteinströmflächen aufweisen. Die Lufteinströmfläche kann insbesondere Bereiche, welche für Luft durchlässig sind, und Bereiche, welche für Luft nicht durchlässig sind, aufweisen. Beispielsweise kann eine Öffnung einen für Luft durchlässigen Bereich bilden. Beispielsweise kann ein Material der Außenfläche, welches die Öffnung begrenzt, einen für Luft nicht durchlässigen Bereich bilden.

Insbesondere können sowohl die Bereiche, welche für Luft durchlässig sind, als auch die Bereiche, welche für Luft nicht durchlässig sind, gleichmäßig über die Lufteinströmfläche und/oder über die Außenfläche verteilt sein. Gemäß einer Ausführungsform der Erfindung ist die gesamte Lufteinströmfläche für Luft im Wesentlichen durchlässig, insbesondere durchlässig. Gemäß einer Ausführungsform der Erfindung bilden die Bereiche, die für Luft durchlässig sind, wenigstens 50 Prozent, insbesondere wenigstens 70 Prozent, bevorzugt wenigstens 90 Prozent der Lufteinströmfläche.

Die Luftausströmfläche kann insbesondere eine Ausblasfläche der Luftkühlung der Gantry bilden. Eine Störung des TAV-Feldes kann insbesondere verringert werden, indem das Ausblasen der Luft möglichst am Rande des TAV-Feldes erfolgt. Beispielsweise kann die Ausblasfläche seitlich an der Gantry oder auf einer Hinterseite der Gantry, welche einem Lagerungssockel der Patientenlagerungsvorrichtung abgewandt ist, angeordnet sein. Alternativ oder zusätzlich kann die Ausblasfläche im unteren Bereich der Gantry angeordnet sein und/oder ein aus der Ausblasfläche ausströmender Luftstrom mittels Lamellen nach unten gerichtet werden.

Ferner kann eine Störung des TAV-Feldes insbesondere dadurch verringert werden, dass eine Außenkontur der Gantry für die TAV strömungsgünstig gestaltet wird. Die Außenkontur der Gantry kann insbesondere derart gestaltet werden, dass in Richtung der TAV Kanten, Vorsprünge oder starke Konturänderungen weitgehend vermieden oder ausgeschlossen werden.

Die erfindungsgemäße Lösung ermöglicht insbesondere, eine Gantry für eine medizinische Bildgebungsvorrichtung bereitzustellen, welche in einem TAV-Feld betrieben werden kann, wobei Luft aus dem TAF-Feld für die Luftkühlung der Gantry verwendet wird, wobei eine Störung des TAV-Feldes weitgehend vermieden wird. Darüber hinaus kann die erfindungsgemäße Gantry in dem TAV-Feld in weiten Bereichen verschoben werden, ohne dass sich dadurch eine Beeinflussung des TAV-Feldes durch die Gantry signifikant ändert.

Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem PET-CT-Gerät und einem PET-MR-Gerät, besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Ohne Einschränkung des allgemeinen Erfindungsgedankens wird bei einigen der Ausführungsformen ein Computertomographiegerät beispielhaft für eine medizinische Bildgebungsvorrichtung genannt.

Gemäß einer Ausführungsform der Erfindung weist die medizinische Bildgebungsvorrichtung eine Akquisitionseinheit, welche zur Akquisition der Akquisitionsdaten ausgebildet ist, auf. Insbesondere kann die Akquisitionseinheit eine Strahlungsquelle und einen Strahlungsdetektor aufweisen. Eine Ausführungsform der Erfindung sieht vor, dass die Strahlungsquelle zur Emission und/oder zur Anregung einer Strahlung, insbesondere einer elektromagnetischen Strahlung, ausgebildet ist und/oder dass der Strahlungsdetektor zur Detektion der Strahlung, insbesondere der elektromagnetischen Strahlung, ausgebildet ist. Die Strahlung kann beispielsweise von der Strahlungsquelle zu einem abzubildenden Bereich gelangen und/oder nach einer Wechselwirkung mit dem abzubildenden Bereich zu dem Strahlungsdetektor gelangen.

Bei der Wechselwirkung mit dem abzubildenden Bereich wird die Strahlung modifiziert und damit zum Träger von Informationen, die den abzubildenden Bereich betreffen. Bei der Wechselwirkung der Strahlung mit dem Detektor werden diese Informationen in Form von Akquisitionsdaten erfasst.

Insbesondere bei einem Computertomographiegerät und bei einem C-Bogen-Röntgengerät können die Akquisitionsdaten Projektionsdaten, die Akquisitionseinheit eine Projektionsdaten-Akquisitionseinheit, die Strahlungsquelle eine Röntgenquelle, der Strahlungsdetektor ein Röntgendetektor sein. Der Röntgendetektor kann insbesondere ein quantenzählender und/oder energieauflösender Röntgendetektor sein.

Insbesondere bei einem Magnetresonanztomographiegerät können die Akquisitionsdaten ein Magnetresonanzdatensatz, die Akquisitionseinheit eine Magnetresonanzdaten-Akquisitionseinheit, die Strahlungsquelle eine erste Hochfrequenzantenneneinheit, der Strahlungsdetektor die erste Hochfrequenzantenneneinheit und/oder eine zweite Hochfrequenzantenneneinheit sein.

Die Gantry einer medizinischen Bildgebungsvorrichtung weist typischerweise eine Tragkonstruktion auf, an der insbesondere Komponenten der Akquisitionseinheit, insbesondere die Strahlungsquelle und/oder der Strahlungsdetektor, angeordnet sind. Die Tragkonstruktion der Gantry weist typischerweise eine derart hohe Steifigkeit und Festigkeit auf, dass die Komponenten der Akquisitionseinheit sowohl relativ zueinander als auch relativ zu einem abzubildenden Bereich in einer für die Bildgebung hinreichend definierten Geometrie anordenbar sind. Bei einem Computertomographiegerät weist die Gantry typischerweise einen Tragrahmen und einen relativ zu dem Tragrahmen drehbar gelagerten Rotor auf, wobei die Strahlungsquelle und der Strahlungsdetektor an dem Rotor angeordnet sind. Optional kann die Gantry einen relativ zu dem Tragrahmen kippbar gelagerten Kipprahmen aufweisen, wobei der Rotor an dem Kipprahmen angeordnet ist.

Bei einem C-Bogen-Röntgengerät weist die Gantry typischerweise einen Tragrahmen und einen relativ zu dem Tragrahmen schwenkbar gelagerten C-Bogen auf, wobei die Strahlungsquelle und der Strahlungsdetektor an dem C-Bogen angeordnet sind.

Bei einem Magnetresonanztomographiegerät weist die Gantry typischerweise einen Tragrahmen auf, an dem der Hauptmagnet und eine erste Hochfrequenzantenneneinheit angeordnet sind, wobei die erste Hochfrequenzantenneneinheit in Form einer Körperspule, die dem Fachmann auch unter dem Begriff "Body Coil" bekannt ist, ausgebildet ist.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der ein Verfahren betrifft, auch mit Merkmalen, die im Zusammenhang mit einer Vorrichtung beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
- Fig. 1: eine schematische Ansicht eines Ausführungsbeispiels einer erfindungsgemäßen Gantry für eine medizinische Bildgebungsvorrichtung,
- Fig. 2: eine schematische Ansicht eines Ausführungsbeispiels einer erfindungsgemäßen Gantry für eine medizinische Bildgebungsvorrichtung,
- Fig. 3: eine schematische Ansicht eines Ausführungsbeispiels einer erfindungsgemäßen Gantry in einer Orthogonalprojektion von oben,
- Fig. 4: eine schematische Ansicht eines Ausführungsbeispiels einer erfindungsgemäßen Anordnung,
- Fig. 5: eine schematische Ansicht einer herkömmlichen Gantry in einem TAV-Feld,
- Fig. 6: ein Blockschema eines Ausführungsbeispiels eines erfindungsgemäßen Verfahrens zur Luftkühlung,
- Fig. 7: eine schematische Ansicht eines Ausführungsbeispiels einer erfindungsgemäßen medizinischen Bildgebungsvorrichtung.

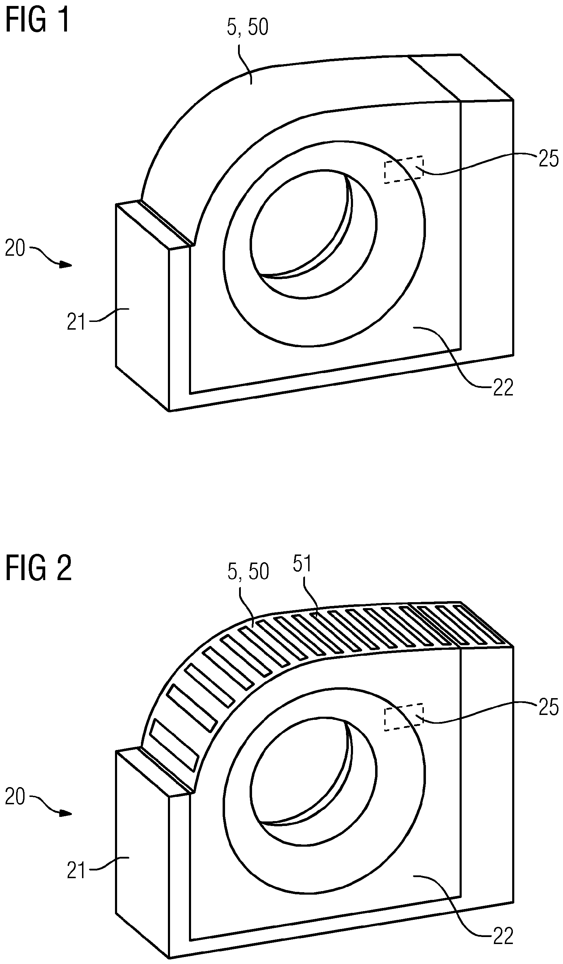

Das in Fig. 1 gezeigte Ausführungsbeispiel einer erfindungsgemäßen Gantry 20 für eine medizinische Bildgebungsvorrichtung 1 weist eine Außenfläche 5 auf. Die Außenfläche 5 kann insbesondere von einer Verkleidung der Gantry 20 gebildet sein. Die Verkleidung kann beispielsweise auf einer Tragkonstruktion montiert sein und/oder selbsttragend, insbesondere selbst eine Tragkonstruktion bildend, ausgebildet sein.

In der Außenfläche 5 der Gantry 20 ist eine Lufteinströmfläche 50 für eine Luftkühlung zumindest einer Komponente 25 der Gantry 20 ausgebildet. In dem Betriebszustand der Gantry 20, der in der Fig. 1 gezeigt ist, ist die Gantry 20 in einem Bereich der Lufteinströmfläche 50 durch die Lufteinströmfläche 50 nach oben begrenzt. Die Gantry 20 ist im Wesentlichen durch die Lufteinströmfläche 50 nach oben begrenzt. Bei der zumindest einen Komponente 25 kann es sich beispielsweise um eine Strahlungsquelle 26 und/oder um einen Detektor 28 handeln.

Das in Fig. 1 gezeigte Ausführungsbeispiel einer erfindungsgemäßen Gantry 20 für eine medizinische Bildgebungsvorrichtung 1 weist eine Lufteinströmfläche 50 mit Lufteinströmelementen 51 in Form von Lufteinlaufspalten auf. Durch die Anordnung der Lufteinlaufspalte kann eine gleichmäßige Lufteinströmgeschwindigkeit realisiert werden, insbesondere derart, dass eine vertikale Komponente der Lufteinströmgeschwindigkeit der Strömungsgeschwindigkeit des laminaren Luftstroms 55 entspricht. Der Abstand zwischen zwei unmittelbar benachbarten Lufteinlaufspalten ist in Bereichen, in denen die Außenfläche 5 der Gantry 20 eine starke Neigung aufweist, größer als in Bereichen, in denen die Außenfläche 5 der Gantry 20 eine geringe Neigung aufweist.

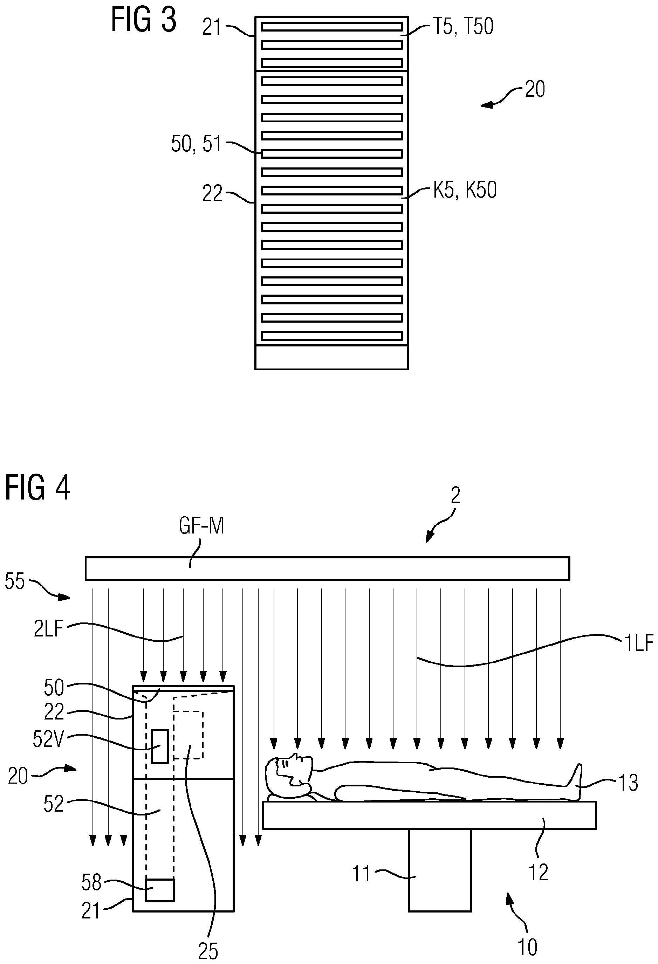

In der Orthogonalprojektion von oben in eine horizontale Ebene, die in der Fig. 3 gezeigt ist, bildet die Lufteinströmfläche 50 im Wesentlichen die gesamte Außenfläche 5 der Gantry 20. Die Lufteinströmfläche 50 nimmt den insbesondere vertikal von oben auf die Gantry 20 strömenden Gantry-Teilluftstrom 2LF des laminaren Luftstroms 55 gleichmäßig verteilt über den im Wesentlichen gesamten Teil der Außenfläche 5, welcher die Gantry 20 nach oben begrenzt, auf. In der dargestellten Orthogonalprojektion ist der Abstand zwischen unmittelbar benachbarten Lufteinlaufspalten im Wesentlichen gleich für alle Lufteinlaufspalte.

Die in die horizontale projizierte Lufteinströmfläche 50 weist eine mittlere Lufteinströmgeschwindigkeit von 0,2 bis 0,3 Metern pro Sekunde auf. Die Kühlluftmenge, welche durch den Kühlkanal 52 strömt, entspricht dem Volumenstrom, der sich ergibt, wenn die Fläche der Orthogonalprojektion der Lufteinströmfläche 50 von oben in eine horizontale Ebene mit der Lufteinströmgeschwindigkeit multipliziert wird.

Die Gantry 20 kann insbesondere derart ausgebildet sein, dass diese Kühlluftmenge für die Luftkühlung der zumindest einen Komponente bei einer typischen Auslastung und/oder bei einer maximalen Auslastung der zumindest einen Komponente ausreicht. Insbesondere kann die medizinische Bildgebungsvorrichtung 1 derart ausgebildet sein, dass der zumindest eine Gantry-Teilluftstrom 2LF des laminaren Luftstroms 55 kontinuierlich in die Gantry 20 einströmt, insbesondere auch dann, wenn sich die medizinische Bildgebungsvorrichtung 1 in einem Ruhezustand (Stand by - Zustand) befindet.

Fig. 4 zeigt eine schematische Ansicht eines Ausführungsbeispiels einer erfindungsgemäßen Anordnung 2. Die Anordnung 2 weist die Gantry 20 und eine Luftstromerzeugungseinheit GF-M auf. Die Luftstromerzeugungseinheit GF-M erzeugt den laminaren Luftstroms 55 derart, dass zumindest ein Gantry-Teilluftstrom 2LF des laminaren Luftstroms 55 von oben auf die Gantry 20 strömt und durch die Lufteinströmfläche 50 in die Gantry 20 hineinströmt.

Die Gantry 20 weist einen Kühlkanal 52 für die Luftkühlung der zumindest einen Komponente 25 der Gantry 20 auf, wobei die Lufteinströmfläche 50 in dem Betriebszustand der Gantry 20 derart ausgebildet ist, dass ein von oben auf die Gantry 20 strömender laminarer Luftstrom 55 durch die Lufteinströmfläche 50 turbulenzarm in den Kühlkanal 52 einströmen kann. Dadurch ist der Verdrängungseffekt der Gantry auf das TAV-Feld 55 relativ gering. In dem Kühlkanal 52 befindet sich eine Luftpumpe 52, die in dem Bereich der Lufteinströmfläche 50 Kühlluft in den Kühlkanal 52 einsaugen kann und/oder in dem Bereich der Luftausströmfläche 58 die erwärmte Kühlluft ausblasen kann. Der Kühlkanal 52 ist in einem an die Lufteinströmfläche 50 anschließenden Bereich trichterförmig erweitert. Die Lufteinströmfläche 50 kann somit auch als Luftansaugfläche bezeichnet werden.

Die Anordnung weist ferner eine Patientenlagerungsvorrichtung 10 der medizinischen Bildgebungsvorrichtung 1 auf, wobei die Luftstromerzeugungseinheit GF-M zur Erzeugung des laminaren Luftstroms 55 derart ausgebildet ist, dass zumindest ein Patienten-Teilluftstrom 1LF des laminaren Luftstroms 55 insbesondere vertikal von oben auf die Patientenlagerungsvorrichtung 10 strömt.

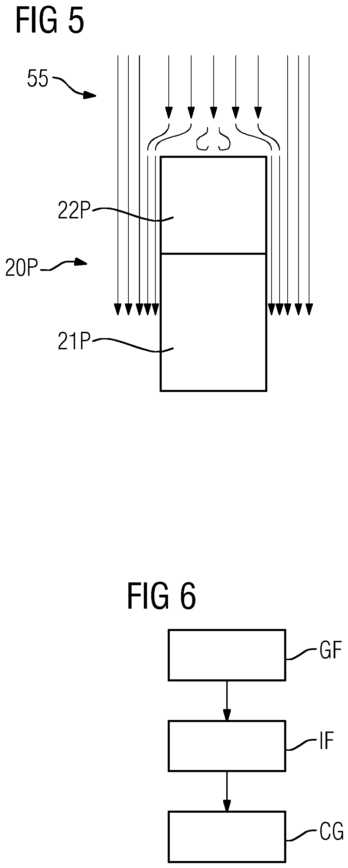

Fig. 5 zeigt eine schematische Ansicht einer herkömmlichen Gantry 20P in einem TAV-Feld 55. In den Bereichen mit eng beieinander liegenden Pfeilen ist auf Grund des Verdrängungseffekts die Strömungsgeschwindigkeit relativ hoch, so dass eine Störung der TAV vorliegt. Eine besonders starke Störung der TAV kann entstehen, wenn die Kühlluft auf der nach oben gerichteten Außenfläche der Gantry ausströmt.

Fig. 6 ein Blockschema eines Ausführungsbeispiels eines erfindungsgemäßen Verfahrens zur Luftkühlung, wobei das Verfahren die folgenden Schritte umfasst:
- Erzeugen GF eines laminaren Luftstroms 55, wobei zumindest ein Gantry-Teilluftstrom 2LF des laminaren Luftstroms 55 von oben auf die Gantry 20 strömt,
- Aufnahme IF des zumindest einen Gantry-Teilluftstroms 2LF in einen Kühlkanal 52 der Gantry 20 über eine Lufteinströmfläche 50, welche an einer Außenfläche 5 der Gantry 20 ausgebildet ist und welche die Gantry 20 in einem Bereich der Lufteinströmfläche 50 nach oben begrenzt, wobei ein Kühlluftstrom CF erzeugt wird, welcher durch den Kühlkanal 52 strömt,
- Kühlen CG der zumindest einen Komponente 25 der Gantry 20 mittels des Kühlluftstroms CF.

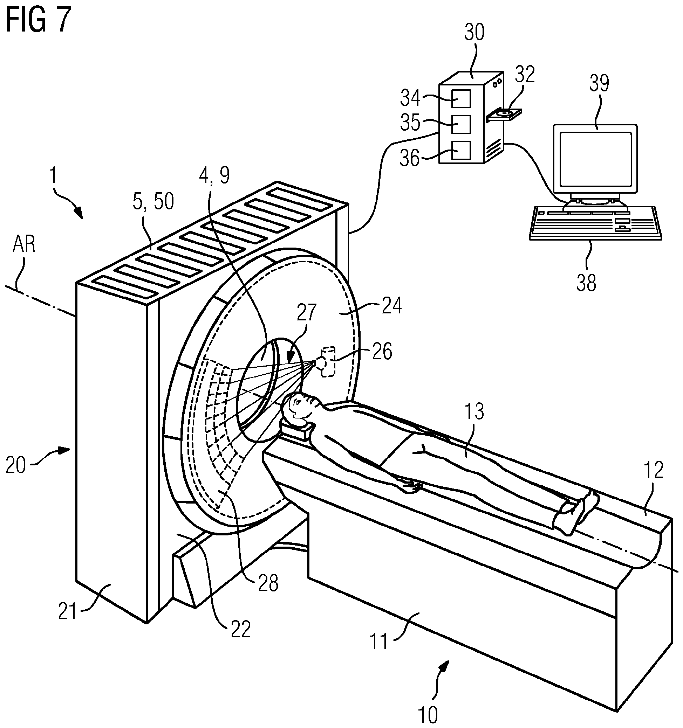

Fig. 7 eine schematische Ansicht eines Ausführungsbeispiels einer erfindungsgemäßen medizinischen Bildgebungsvorrichtung 1. Ohne Beschränkung des allgemeinen Erfindungsgedankens ist für die medizinische Bildgebungsvorrichtung 1 beispielhaft ein Computertomographiegerät gezeigt. Die medizinische Bildgebungsvorrichtung 1 weist die Gantry 20, die tunnelförmige Öffnung 9, die Patientenlagerungsvorrichtung 10 und die Steuerungsvorrichtung 30 auf.

Die Gantry 20 weist den stationären Tragrahmen 21, den Kipprahmen 22 und den Rotor 24 auf. Der Kipprahmen 22 ist mittels einer Kipplagerungsvorrichtung an dem stationären Tragrahmen 21 um eine im Wesentlichen horizontale Kippachse relativ zu dem stationären Tragrahmen 21 kippbar angeordnet. Der Rotor 24 ist mittels einer Drehlagerungsvorrichtung an dem Kipprahmen 22 um eine Rotationsachse AR relativ zu dem Kipprahmen 22 drehbar angeordnet. Die Rotationsachse AR ist im Wesentlichen senkrecht zu der Kippachse und im Wesentlichen parallel zu der Längsrichtung der Lagerungsplatte 12. Der Betriebszustand der Gantry 20, in dem die Gantry 20 in einem Bereich der Lufteinströmfläche 50 durch die Lufteinströmfläche 50 nach oben begrenzt ist, kann beispielsweise ein Betriebszustand sein, in dem die Rotationsachse AR im Wesentlichen horizontal, insbesondere horizontal ist. Bei der Außenfläche 5 der Gantry 20 kann es sich insbesondere um eine Außenfläche 5 der Gantry 20 handeln, welche der tunnelförmigen Öffnung 9 abgewandt ist.

In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist.

Die medizinische Bildgebungsvorrichtung 1 ist zur Akquisition von Akquisitionsdaten basierend auf einer elektromagnetischen Strahlung 27 ausgebildet. Die medizinische Bildgebungsvorrichtung 1 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor. Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 1 ausgebildet. Insbesondere kann auch die Luftstromerzeugungseinheit GF-M mittels der Steuerungsvorrichtung 30 gesteuert werden. Die Steuerungsvorrichtung 30 weist die Datenverarbeitungseinheit 35, das computerlesbare Medium 32 und das Prozessorsystem 36 auf. Die Steuerungsvorrichtung 30, insbesondere die Datenverarbeitungseinheit 35, wird von einem Datenverarbeitungssystem, welches einen Computer aufweist, gebildet.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden. Die medizinische Bildgebungsvorrichtung 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

## Patentansprüche

1. Gantry (20) für eine medizinische Bildgebungsvorrichtung (1),
- wobei in einer Außenfläche (5) der Gantry (20) eine Lufteinströmfläche (50) für eine Luftkühlung zumindest einer Komponente (25) der Gantry (20) ausgebildet ist,
- wobei in einem Betriebszustand der Gantry (20) die Gantry (20) in einem Bereich der Lufteinströmfläche (50) durch die Lufteinströmfläche (50) nach oben begrenzt ist,
- **dadurch gekennzeichnet, dass** die Gantry (20) einen stationären Tragrahmen (21) und einen Kipprahmen (22) aufweist,
- wobei die Außenfläche (5) in einem Bereich des Tragrahmens (21) eine Tragrahmen-Außenfläche (T5) aufweist,
- wobei die Außenfläche (5) in einem Bereich des Kipprahmens eine Kipprahmen-Außenfläche (K5) aufweist,
- wobei eine Tragrahmen-Teilfläche (T50) der Lufteinströmfläche (50) in der Tragrahmen-Außenfläche (K50) ausgebildet ist und/oder wobei eine Kipprahmen-Teilfläche (K50) der Lufteinströmfläche (50) in der Kipprahmen-Außenfläche (K5) ausgebildet ist.

2. Gantry (20) nach Anspruch 1,
- wobei in dem Betriebszustand der Gantry (20) die Gantry (20) im Wesentlichen durch die Lufteinströmfläche (50) nach oben begrenzt ist.

3. Gantry (20) nach Anspruch 1 oder 2,
- wobei in einer Orthogonalprojektion von oben die Lufteinströmfläche (50) wenigstens 50 Prozent der Außenfläche (5) der Gantry (20) bildet.

4. Gantry (20) nach einem der Ansprüche 1 bis 3,
- wobei die Gantry (20) einen Kühlkanal (52) für die Luftkühlung der zumindest einen Komponente (25) der Gantry (20) aufweist,
- wobei die Lufteinströmfläche (50) in dem Betriebszustand der Gantry (20) derart ausgebildet ist, dass ein von oben auf die Gantry (20) strömender laminarer Luftstrom (55) durch die Lufteinströmfläche (50) turbulenzarm in den Kühlkanal (52) einströmen kann.

5. Gantry (20) nach einem der Ansprüchen 1 bis 4,
- wobei die Lufteinströmfläche (50) für eine mittlere Lufteinströmgeschwindigkeit von 0,2 bis 0,3 Metern pro Sekunde ausgebildet ist.

6. Gantry (20) nach einem der Ansprüche 1 bis 5,
- wobei eine flächige Verteilung eines Lufteinströmparameters in der Lufteinströmfläche (50) derart an eine Neigung der Außenfläche (5) der Gantry (20) angepasst ist, dass der Lufteinströmparameter in einer Orthogonalprojektion von oben eine im Wesentlichen gleichmäßige flächige Verteilung aufweist.

7. Gantry (20) nach einem der Ansprüche 1 bis 5,
- wobei die Lufteinströmfläche (50) ein Lufteinströmelement (51) aufweist, welches aus der Gruppe gewählt ist, welche aus einem Luftfilter, einer Lochblende, einer Einströmlamelle und Kombinationen davon besteht.

8. Gantry (20) nach einem der Ansprüche 6 oder 7,
- wobei der Lufteinströmparameter aus der Lufteinströmparametergruppe gewählt ist, welche aus einem Druckverlustbeiwert, einem Öffnungsabstand, einer Öffnungsfläche und Kombinationen davon besteht.

9. Gantry (20) nach einem der Ansprüche 1 bis 8,
- wobei die Gantry (20) eine Luftausströmfläche (58) für die Luftkühlung der zumindest einen Komponente (25) der Gantry (20) aufweist,
- wobei die Luftausströmfläche (58) und die Lufteinströmfläche (50) mittels eines Kühlkanals (52) miteinander verbunden sind.

10. Medizinische Bildgebungsvorrichtung (1), aufweisend eine Gantry (20) nach einem der Ansprüche 1 bis 9.

11. Anordnung (2), aufweisend eine Gantry (20) gemäß einem der Ansprüche 1 bis 9 und eine Luftstromerzeugungseinheit (GF-M),
- wobei sich die Gantry (20) in einem Betriebszustand der Gantry (20) befindet, in dem die Gantry (20) in dem Bereich der Lufteinströmfläche (50) durch die Lufteinströmfläche (50) nach oben begrenzt ist,
- wobei die Luftstromerzeugungseinheit (GF-M) zur Erzeugung des laminaren Luftstroms (55) derart ausgebildet ist,
- dass zumindest ein Gantry-Teilluftstrom (2LF) des laminaren Luftstroms (55) von oben auf die Gantry (20) strömt und durch die Lufteinströmfläche (50) in die Gantry (20) hineinströmt.

12. Anordnung (2) nach Anspruch 11, ferner aufweisend eine Patientenlagerungsvorrichtung (10) der medizinischen Bildgebungsvorrichtung (1),
- wobei die Luftstromerzeugungseinheit (GF-M) zur Erzeugung des laminaren Luftstroms (55) derart ausgebildet ist,
- dass zumindest ein Patienten-Teilluftstrom (1LF) des laminaren Luftstroms (55) von oben auf die Patientenlagerungsvorrichtung (10) strömt.

13. Anordnung (2) nach Anspruch 11 oder 12, ferner aufweisend die medizinische Bildgebungsvorrichtung (1).

14. Verfahren zur Luftkühlung zumindest einer Komponente (25) einer Gantry (20) nach einem der Ansprüche 1 bis 9 einer medizinischen Bildgebungsvorrichtung (1), wobei das Verfahren die folgenden Schritte umfasst:
- Erzeugen (GF) eines laminaren Luftstroms (55), wobei zumindest ein Gantry-Teilluftstrom (2LF) des laminaren Luftstroms (55) von oben auf die Gantry (20) strömt,
- Aufnahme (IF) des zumindest einen Gantry-Teilluftstroms (2LF) in einen Kühlkanal (52) der Gantry (20) über eine Lufteinströmfläche (50), welche an einer Außenfläche (5) der Gantry (20) ausgebildet ist und welche die Gantry (20) in einem Bereich der Lufteinströmfläche (50) nach oben begrenzt, wobei ein Kühlluftstrom (CF) erzeugt wird, welcher durch den Kühlkanal (52) strömt,
- Kühlen (CG) der zumindest einen Komponente (25) der Gantry (20) mittels des Kühlluftstroms (CF).

## Claims

1. Gantry (20) for a medical imaging facility (1),
- wherein an air inflow surface (50) for air cooling of at least one component (25) of the gantry (20) is embodied in an outer surface (5) of the gantry (20),
- wherein, in an operating state of the gantry (20), the gantry (20) is delimited to the top in an area of the air inflow surface (50) by the air inflow surface (50),
- **characterised in that** the gantry (20) has a stationary support frame (21) and a tilting frame (22),
- wherein the outer surface (5), in an area of the support frame (21), has a support frame outer surface (T5),
- wherein the outer surface (5), in an area of the tilting frame, has a tilting frame outer surface (K5),
- wherein a support frame part surface (T50) of the air inflow surface (50) is embodied in the support frame outer surface (K50) and/or wherein a tilting frame part surface (K50) of the air inflow surface (50) is embodied in the tilting frame outer surface (K5).

2. Gantry (20) according to claim 1,
- wherein, in the operating state of the gantry (20), the gantry (20) is essentially delimited to the top by the air inflow surface (50).

3. Gantry (20) according to claim 1 or 2,
- wherein, in an orthogonal projection from above, the air inflow surface (50) forms at least 50 percent of the outer surface (5) of the gantry (20).

4. Gantry (20) according to one of claims 1 to 3,
- wherein the gantry (20) has a cooling duct (52) for air cooling of the at least one component (25) of the gantry (20),
- wherein the air inflow surface (50), in the operating state of the gantry (20), is embodied such that a laminar flow of air (55) flowing onto the gantry (20) from above can flow into the cooling duct (52) through the air inflow surface (50) with low turbulence.

5. Gantry (20) according to one of claims 1 to 4,
- wherein the air inflow surface (50) is embodied for an average air inflow speed of 0.2 to 0.3 meters per second.

6. Gantry (20) according to one of claims 1 to 5,
- wherein a planar distribution of an air inflow parameter in the air inflow surface (50) is adapted to a slope of the outer surface (5) of the gantry (20) such that the air inflow parameter, in an orthogonal projection from above, has an essentially even planar distribution.

7. Gantry (20) according to one of claims 1 to 5,
- wherein the air inflow surface (50) has an air inflow element (51), which is selected from the group that consists of an air filter, an aperture plate, an inflow slat and combinations thereof.

8. Gantry (20) according to one of claims 6 or 7,
- wherein the air inflow parameter is selected from the air inflow parameter group that consists of a pressure loss coefficient, an opening spacing, an opening surface and combinations thereof.

9. Gantry (20) according to one of claims 1 to 8,
- wherein the gantry (20) has an air outflow surface (58) for the air cooling of the at least one component (25) of the gantry (20),
- wherein the air outflow surface (58) and the air inflow surface (50) are connected to one another by means of a cooling duct (52).

10. Medical imaging facility (1), having a gantry (20) according to one of claims 1 to 9.

11. Arrangement (2), having a gantry (20) according to one of claims 1 to 9 and an airflow generation unit (GF-M),
- wherein the gantry (20) is in an operating state of the gantry (20) in which the gantry (20) is delimited to the top in the area of the air inflow surface (50) by the air inflow surface (50),
- wherein the airflow generation unit (GF-M), to generate the laminar air flow (55), is embodied such that,
- at least one gantry part air flow (2LF) of the laminar air flow (55) flows from above onto the gantry (20) and flows into the gantry (20) through the air inflow surface (50).

12. Arrangement (2) according to claim 11, further having a patient support facility (10) of the medical imaging facility (1),
- wherein the airflow generation unit (GF-M) for generating the laminar air flow (55) is embodied such that,
- at least one patient part air flow (1LF) of the laminar air flow (55) flows from above onto the patient support facility (10) .

13. Arrangement (2) according to claim 11 or 12, further having the medical imaging facility (1).

14. Method for air cooling of at least one component (25) of a gantry (20) according to one of claims 1 to 9 of a medical imaging facility (1), wherein the method comprises the following steps:
- Generation (GF) of a laminar air flow (55), wherein at least one gantry part air flow (2LF) of the laminar air flow (55) flows from above onto the gantry (20),
- Receiving (IF) of the at least one gantry part air flow (2LF) into a cooling duct (52) of the gantry (20) via an air inflow surface (50), which is embodied on an outer surface (5) of the gantry (20) and which delimits the gantry (20) to the top in an area of the air inflow surface (50), wherein a flow of cooling air (CF) is generated, which flows through the cooling duct (52),
- Cooling (CG) of the at least one component (25) of the gantry (20) by means of the cooling air flow (CF).

## Revendications

1. Portique (20) d'une installation (1) d'imagerie médicale,
- dans lequel il est constitué, dans une surface (5) extérieure du portique (20), une surface (50) d'entrée d'air pour un refroidissement par de l'air d'au moins un élément (25) du portique (20),
- dans lequel, dans un état de fonctionnement du portique (20), le portique (20) est délimité vers le haut dans une région de la surface (50) d'entrée d'air par la surface (50) d'entrée d'air,
- **caractérisé en ce que** le portique (20) a un cadre (21) porteur fixe et un cadre (22) basculant,
- dans lequel la surface (5) extérieure a, dans une région du cadre (21) porteur, une surface (T5) extérieure-cadre porteur,
- dans lequel la surface (5) extérieure a, dans une région du cadre basculant, une surface (K5) extérieure-cadre basculant,
- dans lequel une surface (T50) partielle-cadre porteur de la surface (50) d'entrée d'air est constituée dans la surface (K50) extérieure-cadre porteur et/ou dans lequel une surface (K50) partielle-cadre basculant de la surface (50) d'entrée d'air est constituée dans la surface (K5) extérieure-cadre basculant.

2. Portique (20) suivant la revendication 1,
- dans lequel, dans l'état de fonctionnement du portique (20), le portique (20) est délimité vers le haut essentiellement par la surface (50) d'entrée d'air.

3. Portique (20) suivant la revendication 1 ou 2,
- dans lequel, en projection orthogonale du haut, la surface (50) d'entrée d'air forme au moins 50 pour cent de la surface (5) extérieure du portique (20).

4. Portique (20) suivant l'une des revendications 1 à 3,
- dans lequel le portique (20) a un canal (52) de refroidissement pour le refroidissement par de l'air du au moins un élément (25) du portique (20),
- dans lequel la surface (50) d'entrée d'air, dans l'état de fonctionnement du portique (20), est constituée de manière à ce qu'un courant (55) d'air laminaire, s'écoulant du haut sur le portique (20), puisse entrer dans le canal (52) de refroidissement avec peu de turbulence par la surface (50) d'entrée d'air.

5. Portique (20) suivant l'une des revendications 1 à 4,
- dans lequel la surface (50) d'entrée d'air est constituée pour une vitesse moyenne d'écoulement de l'air de 0,2 à 0,3 mètres à la seconde.

6. Portique (20) suivant l'une des revendications 1 à 5, dans lequel une répartition en surface d'un paramètre d'entrée de l'air dans la surface (50) d'entrée d'air est adaptée à une inclinaison de la surface (5) extérieure du portique (20), de manière à ce que le paramètre d'entrée de l'air, en une projection orthogonale du haut, ait une répartition en surface sensiblement uniforme.

7. Portique (20) suivant l'une des revendications 1 à 5,
- dans lequel la surface (50) d'entrée d'air a un élément (51) d'entrée d'air, qui est choisi dans le groupe constitué d'un filtre à air, d'un diaphragme à trou, d'une lamelle d'entrée et de leurs combinaisons.

8. Portique (20) suivant l'une des revendications 6 ou 7,
- dans lequel le paramètre d'entrée de l'air est choisi dans le groupe de paramètres d'entrée de l'air, constitué d'une contribution à la perte de charge, d'une distance d'ouverture, d'une surface d'ouverture et de leurs combinaisons.

9. Portique (20) suivant l'une des revendications 1 à 8,
- dans lequel le portique (20) a une surface (58) de sortie d'air pour le refroidissement par de l'air du au moins un élément (25) du portique (20),
- dans lequel la surface (58) de sortie d'air et la surface (50) d'entrée d'air communiquent entre elles au moyen d'un conduit (52) de refroidissement.

10. Installation (1) d'imagerie médicale ayant un portique (20) suivant l'une des revendications 1 à 9.

11. Système (2) ayant un portique (20) suivant l'une des revendications 1 à 9 et une unité (GF-M) de production d'un courant d'air,
- dans lequel le portique (20) se trouve dans un état de fonctionnement, dans lequel le portique (20) est, dans la région de la surface (50) d'entrée d'air, délimité vers le haut par la surface (50) d'entrée d'air,
- dans lequel l'unité (GF-M) de production d'un courant d'air est constituée pour la production du courant (55) d'air laminaire,
- en ce qu'au moins un courant (2LF) d'air partiel de portique du courant (55) d'air laminaire s'écoule du haut sur le portique (20) et passe par la surface (50) d'entrée d'air dans le portique (20).

12. Système (2) suivant la revendication 11, comportant, en outre, un dispositif (10) de couchette de patient de l'installation (1) d'imagerie médicale,
- dans lequel l'unité (GF-M) de production d'un courant d'air est constituée pour la production du courant (55) d'air laminaire, de manière,
- à ce qu'au moins un courant (1LF) d'air partiel de patient du courant (55) d'air laminaire passe du haut sur le dispositif (10) de couchette du patient.

13. Système (2) suivant la revendication 11 ou 12, comportant, en outre, l'installation (1) d'imagerie médicale.

14. Procédé de refroidissement par de l'air d'au moins un élément (25) d'un portique (20) suivant l'une des revendications 1 à 9 d'une installation (1) d'imagerie médicale, le procédé comprenant les stades suivants :
- la production (GF) d'un courant (55) d'air laminaire, au moins un courant (2LF) d'air partiel de portique du courant (55) d'air laminaire passant du haut sur le portique (20),
- la réception (IF) du au moins un courant (2LF) d'air partiel de portique dans un conduit (52) de refroidissement du portique (20) par l'intermédiaire d'une surface (50) d'entrée d'air, qui est constituée sur une surface (5) extérieure du portique (20) et qui délimite vers le haut le portique (2) dans une région de la surface (50) d'entrée d'air, un courant (CF) d'air de refroidissement étant produit, qui passe dans le conduit (52) de refroidissement,
- le refroidissement (CG) du au moins un élément (25) du portique (20) au moyen du courant (CF) d'air de refroidissement.
